# EUROPEAN PATENT SPECIFICATION

(11) **EP 4 627 349 B1**
(45) Date of publication and mention of the grant of the patent: **05.08.2026**
(21) Application number: 23813767.3
(22) Date of filing: 30.11.2023
(51) Int. Cl.: G16B 40/10, G01N 33/58, G01N 33/68, G16B 20/00

(54) **BIOMOLECULE ANALYSIS BY FLUORESCENCE INTERMITTENCE SIGNAL RECOGNITION**
BIOMOLEKÜLANALYSE MITTELS ERKENNUNG VON INTERMITTIERENDEN FLUORESZENZSIGNALEN
ANALYSE BIOMOLECULAIRE PAR RECONNAISSANCE DE SIGNAL D'INTERMITE DE FLUORESCENCE

(30) Priority: 30.11.2022 EP 22210671
(43) Date of publication of application: 08.10.2025
(73) Proprietor: Universität Zürich, 8006 Zürich (CH)
(72) Inventor: PÜNTENER, Salome, 8006 Zürich (CH); RIVERA FUENTES, Pablo, 8006 Zürich (CH)
(74) Representative: Schulz Junghans Patentanwälte PartGmbB
(86) International application number: PCT/EP2023/083658
(87) International publication number: WO 2024/115628

(56) References cited:
- DE LANNOY CARLOS VICTOR ET AL: "Evaluation of FRET X for single-molecule protein fingerprinting", ISCIENCE, vol. 24, no. 11, 19 November 2021 (2021-11-19), US, pages 103239 - 103239, XP093045768, ISSN: 2589-0042, DOI: 10.1016/j.isci.2021.103239
- ZHENG QINSI ET AL: "Rational Design of Fluorogenic and Spontaneously Blinking Labels for Super-Resolution Imaging", vol. 5, no. 9, 25 September 2019 (2019-09-25), pages 1602 - 1613, XP093001259, ISSN: 2374-7943, Retrieved from the Internet <URL:http://pubs.acs.org/doi/pdf/10.1021/acscentsci.9b00676> DOI: 10.1021/acscentsci.9b00676
- CHIEN FAN-CHING ET AL: "Single-Molecule Blinking Fluorescence Enhancement by Surface Plasmon-Coupled Emission-Based Substrates for Single-Molecule Localization Imaging", ANALYTICAL CHEMISTRY, vol. 93, no. 46, 3 November 2021 (2021-11-03), US, pages 15401 - 15411, XP093045378, ISSN: 0003-2700, DOI: 10.1021/acs.analchem.1c03206
- PÜNTENER SALOME ET AL: "Single-Molecule Peptide Identification Using Fluorescence Blinking Fingerprints", vol. 145, no. 2, 18 January 2023 (2023-01-18), pages 1441 - 1447, XP093045368, ISSN: 0002-7863, Retrieved from the Internet <URL:https://www.ncbi.nlm.nih.gov/pmc/articles/PMC9853850/pdf/ja2c12561.pdf> DOI: 10.1021/jacs.2c12561

## Description

### Field

The present invention relates to methods and systems for detecting small variations of structure among a plurality of variants of a biomolecule by measuring the blinking pattern of a dye molecule bound to the biomolecules. The method of the invention provides a single-molecule proteomics protocol that can differentiate between biomolecules such as peptides, which differ only by subtle structural modifications, such as post-translational modifications. The scope of protection is defined by the appended claims.

### Background

The diversity of the proteome is only partially determined by the genome. Protein abundance, occurrence of isoforms, and post-translational modifications (PTMs) cannot be predicted from genomic or transcriptomic information. Mass spectrometry-based proteomics methods remain limited by their sensitivity and dynamic range compared to established single-molecule approaches in nucleic acid analysis. In particular, single-molecule identification of peptides and proteins would enable the analysis of biomarkers that are present in very small quantities, for example in diluted clinical samples, single cells, or isolated organelles.

The ability to identify peptides with single-molecule sensitivity would lead to next-generation proteomics methods for biological research and clinical applications. Existing single-molecule peptide sequencing methods can read some amino acid sequences, but they are limited by their ability to distinguish between similar amino acids or post-translational modifications. Recently, significant progress has been made toward single-molecule proteomics. Approaches based on biological and solid-state nanopores, tunneling conductance measurements, N-terminal amino-acid binding probes, single-molecule Edman sequencing, and mass spectrometry hold great potential, but also face challenges, including but not limited to amino acid detection accuracy, difficulties linearizing large peptides and proteins, handling positively charged peptides, and throughput. Single-molecule fluorescence-based techniques are massively parallelizable, but existing methods rely on multiple cycles of chemical or enzymatic degradation (Reed et al., bioRxiv, doi:10.1101/2022.01.04.475002; Swaminathan et al., Nat. Biotechnol. 36, 1076-1091 (2018)), making data acquisition long and prone to errors.

De Lannoy Carlos Victor et al.: "Evaluation of FRET X for single-molecule protein fingerprinting", iScience, vol. 24, no. 11, 19 November 2021 (2021-11-19), pages 103239-103239 discloses fluorescence-based methods (single molecule FRET) that can detect the position of selected amino acids in single peptide molecules.

Based on the above-mentioned state of the art, the objective of the present invention is to provide means and methods to enable single molecule identification of biomolecules. This objective is attained by the subject-matter of the independent claims of the present specification, with further advantageous embodiments described in the dependent claims, examples, figures and general description of this specification.

### Summary of the Invention

Here, the inventors provide proof of a fundamentally new approach to identify biomolecules, in particular peptides, with single-molecule sensitivity. The method does not rely on sequencing and therefore avoids the need to distinguish each individual component of a modular biomolecule, such as an amino acid within a peptide, with high accuracy. This method holds potential as a widely applicable, rapid, and accurate single-molecule biomolecule fingerprinting technology.

The invention relates to a method for identifying a variant of a biomolecule, particularly a specific variant of a biomolecule among a plurality of variants of the biomolecule, by these steps: A) Acquiring a fluorescence intermittence signal pattern for each variant. Each of the variants is labelled with a blinking fluorophore. B) Determining a fluorescence intermittency signal characteristic for each variant. C) Labelling at least one variant of the biomolecule in a sample with the fluorophore, and acquiring its fluorescence intermittency signal. D) Determining a presence of at least one fluorescence intermittency signal characteristic as determined in step B) in the acquired fluorescence intermittency signals from the sample. E) Establishing the presence, the quantity and/or localisation, of at least one variant of the biomolecule in the sample based on the fluorescence intermittency signal characteristic.

### Terms and definitions

For purposes of interpreting this specification, the following definitions will apply and whenever appropriate, terms used in the singular will also include the plural and vice versa.

The terms "comprising", "having", "containing", and "including", and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a", "or" and "the" include plural referents unless the context clearly dictates otherwise.

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic, and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

The term *spontaneous fluorescence intermittency* in the context of the present specification relates to a fluorescence signal emitted by or recorded from a fluorescence emitter exhibiting fluorescence intermittency.

The term *fluorescence intermittence signal pattern* in the context of the present specification relates to the temporal course of the intermittent fluorescence signal of the fluorescence emitter, particularly wherein each temporal course and thus the pattern is different for independent fluorescence emitters.

The term *fluorescence intermittency signal characteristic* in the context of the present specification relates to any observable of the intermittency signal, particularly wherein the observable comprises at least one statistical value characterizing the fluorescence intermittency.

### Detailed Description of the Invention

In one aspect, the invention relates to a method for identifying the presence and identity of a variant of a biomolecule. Identification in this context may mean that the presence and location of a variant is determined without other distinct variants being present. Likewise, the term may refer to the identification of the specific variant among a plurality of (structurally closely related) variants of the biomolecule.

The method according to the invention comprises the steps of:
Acquisition step: Acquiring (e.g., recording or loading from a digital storage medium) a fluorescence intermittence signal pattern for each variant of the plurality. Acquisition of the fluorescence intermittence signal pattern is performed in a separatable manner, such that for each biomolecule of the plurality, the intermittency signal can be determined. Each of the variants is labelled with the same fluorophore, which exhibits spontaneous fluorescence intermittency upon excitation.

The results obtained by the inventors and outlined in the examples show that there is a pattern that can be extracted using mathematical (e. g., machine learning) algorithms. The signal is not completely random, and hence classifiable.

In a determination step, a fluorescence intermittency signal characteristic is determined for each of said variants labelled with the fluorophore, and associated thereto.

In a sample acquisition step, a sample comprising at least one variant of the plurality of variants of biomolecules is provided, and at least one variant of the biomolecule in the sample is labeled with said fluorophore. A fluorescence intermittency signal is acquired from each of the labelled variants, at least from one of them.

The presence of at least one fluorescence intermittency signal characteristic as determined in step b. is determined in the acquired fluorescence intermittency signals from the sample.

The presence and optionally, the quantity and/or localisation, of at least one variant of the biomolecule in the sample is established based on the fluorescence intermittency signal characteristic determined from the fluorescence intermittency signal acquired from the sample and the fluorescence intermittency signal characteristic determined in the determination step.

A specific biomolecule may be labelled in a sample by a dye molecule labelling reaction, and the specific biomolecule may be detected according to a pre-determined fluorescence intermittency signal characteristic that enables attribution of the fluorescence intermittency signal to that specific molecule, in a sample wherein the dye molecule labelling reaction has also labelled other biomolecules. The labelling of these other biomolecules leads to fluorescence intermittency signals with a different characteristic, from which the method can distinguish the signals originating from the specific biomolecule.

Thereby, the method according to the invention may also be used for biomolecule analysis, particularly protein and peptide analysis. The method of the invention is not necessarily limited to discrimination between closely related variants of the same molecule, but can also be used to detect biomolecules in a sample according to their fluorescence intermittency signal characteristic. The inventors are currently exploring ligations to label cysteine and lysine with two different fluorophores. For cysteine, a simple alkyl iodide (similar to iodoacetamide) may be employed; for lysine, an activated ester (perfluorophenyl ester). It may also be feasible attach the alkyne to the N-terminus, also in a full peptide. Zanon et al. have recently published a study that shows how the skilled person may proceed to select different attachment chemistries for proteome-wide labelling of distinct moieties (Zanon PRA, Yu F, Musacchio P, Lewald L, Zollo M, Krauskopf K, et al. Profiling the proteome-wide selectivity of diverse electrophiles. ChemRxiv. Cambridge: Cambridge Open Engage; 2021).

In certain embodiments, the fluorophore is covalently linked to the biomolecule.

In particular embodiments, the biomolecule is labelled by covalent attachment of a dye molecule.

The method of the invention has been demonstrated in the context of peptides, but is not necessarily limited to peptides. A range of other biomolecules, including carbohydrates, might be substrate for detection in a similar fashion.

In certain particular embodiments, the biomolecule is a peptide.

In certain embodiments, the peptide is composed of proteinogenic amino acids.

In certain embodiments, the biomolecule comprises a thiol group, particularly wherein the biomolecule comprises a cysteine residue, and the fluorophore is covalently linked to the cysteine.

In certain embodiments, the fluorophore exhibiting spontaneous fluorescence intermittency is characterized by blinking in the millisecond time scale. The minimum blinking time determined in the examples is 30 ms and the highest median of the maximal blinking times is around 200 ms. Longer blinks can occur but are rare. In certain embodiments, the fluorophore exhibiting spontaneous fluorescence intermittency is characterized by blinking in the range of 30-500 ms.

In certain embodiments, the fluorophore exhibiting spontaneous fluorescence intermittency is a silicon rhodamine, particularly a hydroxymethyl silicon rhodamine.

Generally, any fluorophore that can thermally/spontaneously switch between a fluorescent and a non-fluorescent form and can be measured with single-molecule sensitivity could be applied in the described method. Currently, fluorophores of the rhodamine class (classical and silicon rhodamines) are described to exhibit such behavior (see the publications cited under "Results and Discussion") by spirocyclizing through their nucleophilic group (demonstrated with hydroxyl- and amide-groups) at the lower ring, however, other combinations of nucleophile and rhodamine core may also lead to spontaneous blinking and thus would be applicable to the method according to the invention.

In certain embodiments, the biomolecule is a peptide, and the variants to be discerned by the method according to the invention differ with regard to their amino acid sequence.

In certain embodiments, the biomolecule is a peptide, and the variants to be discerned by the method according to the invention differ with regard to a post-translational modification of their amino acid sequence, particularly with regard to a post-translational modification selected from the group consisting of epimerization, deamidation, citrullination, phosphorylation, ribosylation, glycosylation, acetylation, methylation, hydroxylation.

In certain particular embodiments, the biomolecule is a peptide, and the variants to be discerned by the method according to the invention differ with regard to their post-translational modification, the post-translational modification being selected from the group consisting of epimerization, deamidation, and citrullination.

In certain particular embodiments, the biomolecule is a peptide, and the variants to be discerned by the method according to the invention differ with regard to conformational changes of the biomolecule due to interactions with other biomolecules or its environment.

In certain embodiments, the biomolecule is anchored to a solid surface.

Wherever alternatives for single separable features are laid out herein as "embodiments", it is to be understood that such alternatives may be combined freely to form discrete embodiments of the invention disclosed herein.

The invention is further illustrated by the following examples and figures, from which further embodiments and advantages can be drawn. These examples are meant to illustrate the invention but not to limit its scope.

### Examples

### Results and Discussion

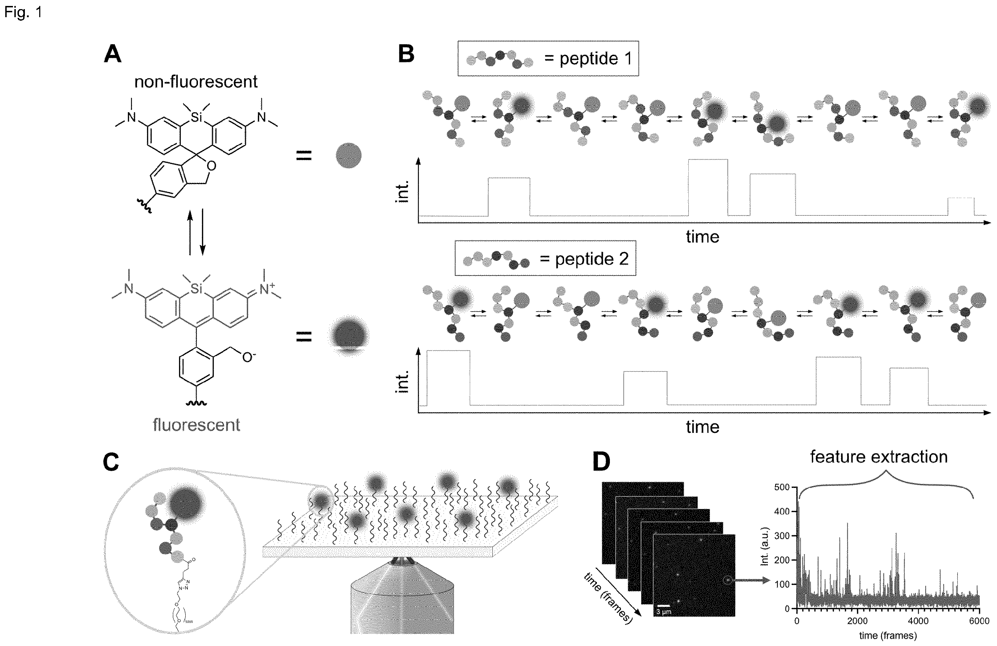

We reasoned that if the fluorescent signal of a labeled biomolecule, particularly a labeled peptide, could encode enough information about its sequence, this fingerprint could be used to identify single molecules composed of a complex mixture of constituent parts, such as a peptide composed of amino acids, without having to determine every single constituent part (i.e., reading every amino acid in the peptide sequence). We hypothesized that a spontaneously blinking fluorophore could capture information about the identity of the whole molecule. These fluorophores have been used for single-molecule localization microscopy because they undergo thermal rearrangements between a fluorescent and a non-fluorescent isomer (Fig. 1A), producing an intermittent pattern of emission (spontaneous blinking). Spontaneously blinking fluorophores include, but are not limited to the molecules discussed in the following publications: Lukinavicius et al., Nature Chemistry 6(8):663-664; Lardon, Wang, Johnsson et al., Systematic Tuning of Rhodamine Spirocyclization for Super-Resolution Microscopy, BioRxiv 2011; Tyson et al., ACS Cent Sci. 2021 Aug 25;7(8):1419-1426; Uno et al., Nat. Chem. 6, 681-689 (2014); Zheng et al., ACS Cent. Sci. 5, 1602-1613 (2019); Halabi et al., Nat. Commun. 10, 1232 (2019); Tachibana et al., Chem. Commun, 56, 13173-13176 (2020); Uno et al., Chem. Commun., 54,102-105 (2018). The lifetimes of the fluorescent and non-fluorescent isomers, and their rate of interconversion, depend on the chemical environment of the probe. Interactions of the fluorophore with the peptide would affect the blinking pattern (Fig. 1B), and therefore information about the structure of the peptide would be encoded in the emission time trace. To measure this blinking pattern, peptides labeled with a spontaneously blinking fluorophore could be attached to a glass surface and imaged by total internal reflection fluorescence (TIRF) microscopy (Fig. 1C). Time-lapse imaging would reveal unique blinking patterns for different peptides, and features could be extracted from these traces to train a machine learning (ML) algorithm to identify the sequence of the peptide (Fig. 1D). We call this approach "blinkognition"; a portmanteau of "blinking" and "recognition".

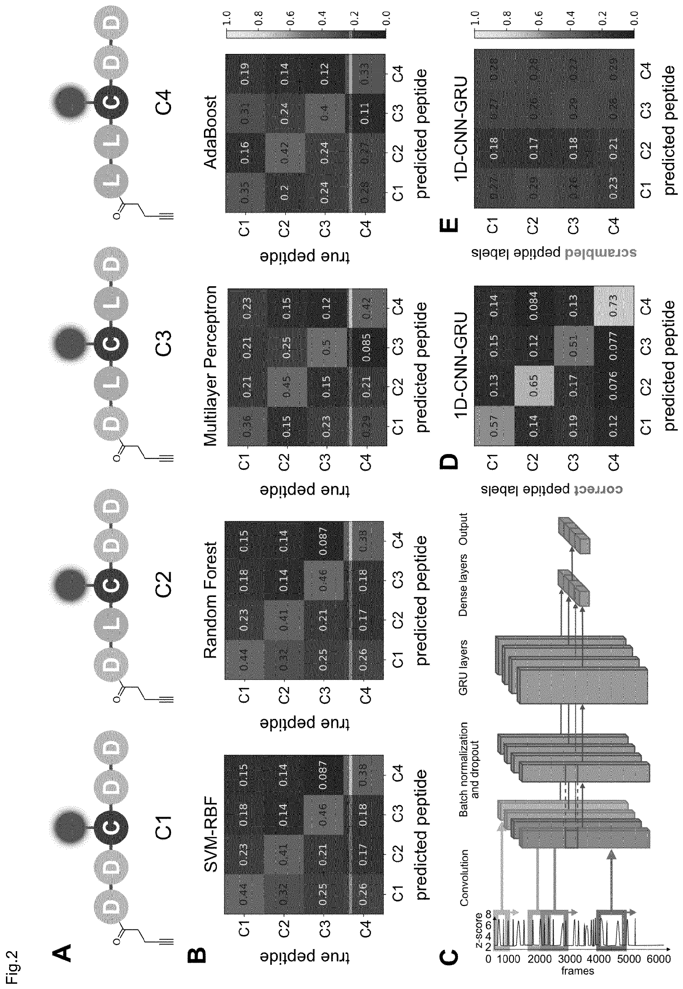

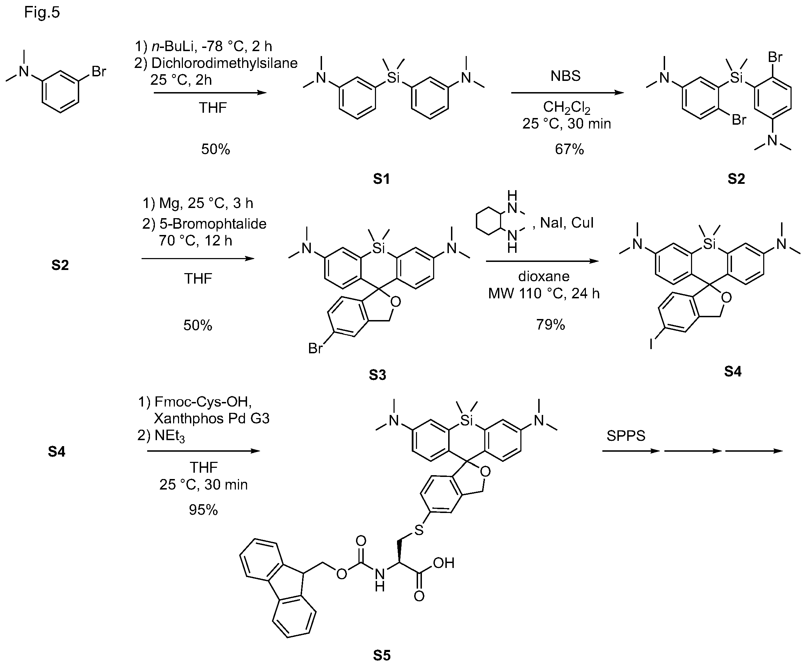

We first tested whether we could identify each molecule within a set of four negatively charged peptides (charged peptide set **C1-C4,** Fig. 2A) based on the spontaneous blinking of an HMSiR fluorophore (Fig. 1A) covalently attached to a cysteine (C) residue within the peptide (Fig. 2A, materials and methods section 1 and Fig. 5). The hypothesis was that these peptides should provide different chemical environments to the fluorophore based on the total number and position of aspartate (D) and leucine (L) residues. Peptides **C1-C4** were synthesized by solid-phase peptide synthesis (materials and methods section 2). To measure their single-molecule fluorescence patterns, we covalently attached the peptides to PEGylated coverslips by click chemistry (materials and methods section 3) and imaged them using TIRF microscopy (Fig. 1C, material and methods section 4). Single-molecule fluorescence time traces were extracted from time-lapse acquisitions (materials and methods section 5, and tables 1 and 2) and the intensity of the traces was normalized (materials and methods section 5). We split these traces into train and test sets, and manually extracted a few features from the traces, including total number of peaks, peak duration, and photobleaching time (full list of features is provided in table 3). These features were used to train classical ML models that were used to determine the sequence of peptides in the test set. These models could identify the molecules only with modest accuracies (40-50%, Fig. 2B), albeit significantly higher than a random guess (25%). Although these results are not accurate enough for practical applications, they demonstrate that interpretable ML models can extract some sequence-related information from blinking patterns, suggesting that blinkognition is a viable strategy for single-molecule peptide fingerprinting.

We posited that our manually extracted features might not contain all the information that contributes to the uniqueness of blinking traces. Thus, we designed a classifier that utilizes a one-dimensional convolutional neural network (1D-CNN) for downsampling and feature extraction directly from blinking traces. These features are subsequently fed into gated recurrent units (GRUs) to capture the information contained in the sequence (Cho et al., On the properties of neural machine translation: encoder-decoder approaches. arXiv:1409.1259v2 [cs.CL]). Finally, the output is passed through fully connected layers that produce a classification output (Fig. 1C, materials and methods section 5 and table 4). This model was able to assign the correct structure to single molecules of **C1-C4** (Fig. 2A) with an overall accuracy of 62% (Fig. 2D). This increase in overall accuracy demonstrates that the convolutional layers are indeed capable of finding relevant features for the classification of peptide identities based on blinkognition. Importantly, when traces were randomly assigned a wrong label (e. g., a trace corresponding to **C1** was labeled as **C2, C3,** or **C4)** and used to train the model, the algorithm was unable to learn any useful information and failed to identify the molecules beyond a random guess (Fig. 2E). This control experiment indicates that the model is actually learning information related to the sequence of the peptide.

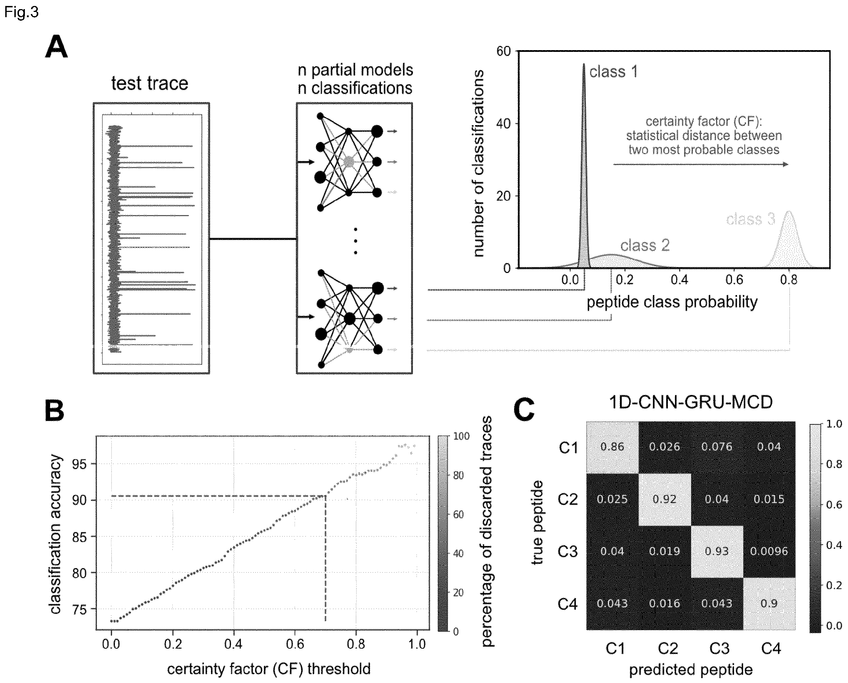

We envisioned that it would be valuable to quantify the certainty with which the model predicts the structure of the peptide. Using a certainty measure, the user could discard low-quality traces to improve the accuracy of the method. This practice is common in DNA sequencing, in which single-molecule signals are often discarded based on the certainty of base prediction. To implement an analogous quality control, we augmented our classifier with Monte Carlo dropout (materials and methods section 5 and table 6) (Gal and Ghahramani, Dropout as a Bayesian approximation: Representing model uncertainty in deep learning. Proc. 33rd Int. Conf. Mach. Learn. 48 (2016)). In this method, random nodes of the neural network are turned off and the resulting partial model is used for prediction. This process is repeated several times, producing a distribution of classification outputs for each input trace. We reasoned that we could use these results to define a classification certainty factor (CF) based on the statistical distance between the distributions of the two most probable peptide classes (Fig. 3A and materials and methods section 5).

We applied the augmented classifier, 1D-CNN-GRU-MCD (materials and methods section 5 and Table 5), to identify peptides **C1-C4.** The CF value lies between 0 and 1, with high numbers indicating high classification certainties. Consequently, when traces with a low CF value were discarded, the overall accuracy of the prediction increased (Fig. 3B). For instance, when we discarded traces with a CF value below 0.7, we could classify peptides **C1-C4** with an overall accuracy of 90% (Fig. 3C). A filtering criterium of CF ≥ 0.7 is relatively strict and about 50% of the measured traces were discarded. In contrast, removing the filter entirely (CF ≥ 0) retains all of the traces but results in a lower overall accuracy of 73%. Thus, the CF threshold can be adapted for applications that require more coverage (low CF) or more accuracy (high CF). Importanly, when we discarded traces randomly, we did not observe any increase in overall accuracy, and filtering by CF value did not seem to bias the composition of the test set significantly. These observations suggest that the CF value is a valid classification certainty indicator that can be used to increase the accuracy of blinkognition.

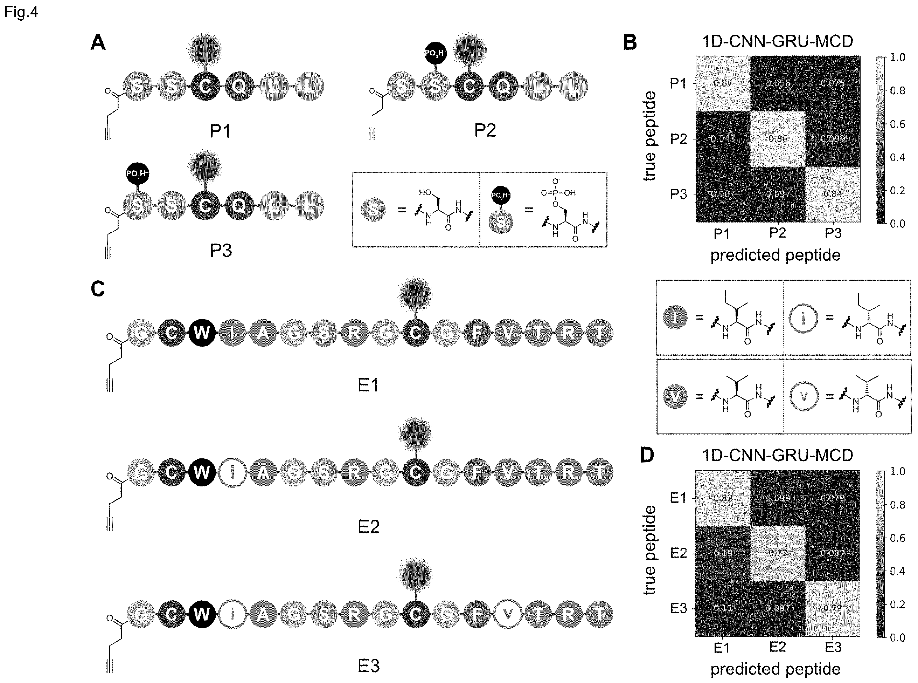

After proving that blinkognition can accurately distinguish between peptides with different sequences, we tested whether we could use it to classify peptides with an identical sequence but that differ by their PTMs. First, we studied the phosphorylation state of the GTP-binding protein Rap1B (Pannekoek et al., Cell Adh. Migr. 8, 100-107 (2014)). This protein is involved in the regulation of endothelial barrier function by modulating cytoskeletal tension. Near the C terminus of Rap1B, serine residues S179 and S180 can be phosphorylated by cAMP-dependent protein kinase A (PKA). Phosphorylation disrupts the interaction with the chaperone SmgGDS-607, preventing prenylation of Rap1B and diminishing its membrane localization. These phosphorylation sites are part of a short peptide (SSCQLL (SEQ ID NO: 5)) that could be obtained by proteolytic cleavage between K178 and S179 in a traditional proteomics experiment. Thus, we synthesized the three relevant peptides (phosphorylation peptide set **P1-P3,** Fig. 4A), and their blinking patterns were measured as described before. Using the 1D-CNN-GRU-MCD model with a CF threshold of 0.7, peptides **P1-P3** could be classified with an overall accuracy of 85% (Fig. 4B). Even at this strict thresholding level, 54% of all measured traces were used. This result demonstrates that blinkognition can be applied robustly to biologically relevant peptides and is sensitive to both the presence and position of PTMs.

Next, we explored whether much more subtle PTMs, such as epimerization, could be detected by peptide blinkognition. For this purpose, we chose OspA, a ribosomally synthesized and post-translationally modified peptide (RiPP) of cyanobacterial origin (*Oscillatoria sp.* PCC 6506) (Freeman et al. Science. 338, 387-390 (2012)). This peptide is consecutively post-translationally epimerized at isoleucine (I4) and valine (V13) residues by the S-adenosyl-L-methionine radical epimerase OspD, to give D-valine (v13) and D-*allo*-isoleucine (i4) residues. We prepared the parent, all-L, peptide and those containing either only i4 or both i4 and v13 (epimerization peptide set **E1-E3,** Fig. 4C) and recorded their blinking patterns as described before. Even in this very challenging peptide fingerprinting case, we could obtain an overall classification accuracy of 79% using a CF threshold of 0.7 (Fig. 4D). This strict thresholding led to a substantial amount of discarded traces (~90%), but as mentioned before, the percentage of retained traces could be increased at the cost of moderate losses in accuracy.

Although enantiomers of isolated amino acids have been identified before by recognition tunneling or using single-molecule junctions, to the best of our knowledge, this is the first example of single-molecule identification of peptides solely differing by a single epimerized residue. Moreover, the fact that these hexadecapeptides **(E1-E3)** are classified with comparable accuracies to penta- and hexapeptides **(C1-C4** and **P1-P3,** respectively) demonstrates that, unlike sequencing methods, the accuracy of blinkognition does not necessarily decrease in longer peptides. Importantly, full sequencing of peptides **E1-E3** would require single-amino-acid accuracies of -98.5% (0.985¹⁶ = 0.79) to achieve the same overall accuracy as blinkognition. To the best of our knowledge, no protein sequencing technique has reached this accuracy for all the amino acids represented in these peptide sets, in particular epimers.

The method described here is suitable for hypothesis-driven studies in which specific peptides are targeted for quantification. Although the accuracy of the method is already high, it could be further improved from both the experimental and analytical fronts, for example by decreasing the noise in training sets, increasing the information content in traces, and applying more sophisticated deep learning models. Several inherent advantages make blinkognition a single-molecule proteomics approach worth developing further. It provides a fast and simple way to detect peptides regardless of their length. Unlike other single-molecule fluorescence approaches, it does not rely on chemical degradation or proteolysis steps. Compared to nanopore sequencing, it does not require denaturation of the peptide and can be applied to both negatively and positively charged peptides (e. g., **C1-C4** and **E1-E3,** respectively). Moreover, this method could be further parallelized to produce million or billions of single-molecule reads, similarly to next-generation DNA sequencing. Finally, we envision that this technique could be applied to other macromolecules for which few analytical techniques exist, such as oligosaccharides. Overall, blinkognition represents a new avenue for the development of single-molecule analytical technologies, which may have a significant impact on both basic research and clinical applications.

### Materials and Methods

### Section 1. Small-molecule synthesis.

A general synthetic scheme is provided in Fig. 5.

**General methods:** All reagents were purchased from commercial sources and used as received. Anhydrous solvents were procured from Acros Organics and used as received. All solvents used in the preparation of the coverslips were HPLC grade. NMR spectra were acquired on Bruker AVANCE NEO-400, Bruker AVANCE III-400, Bruker AVANCE III HD-600, or Bruker AVANCE II-800 instruments. ¹H NMR chemical shifts are reported in ppm relative to SiMe₄ (δ = 0) and were referenced internally with respect to residual protons in the solvent (δ = 7.26 for chloroform, δ = 1.94 for CH₃CN, δ = 3.31 for CH₃OH and δ = 3.58 for THF). Coupling constants are reported in Hz. ¹³C NMR chemical shifts are reported in ppm relative to SiMe₄ (δ = 0) and were referenced internally with respect to solvent signal (δ = 77.16 for CDCl₃ and δ = 1.32 for CD₃CN). Preliminary peak assignments are based on calculated chemical shifts and multiplicity. Low-resolution mass spectra (LRMS) were acquired on a Shimadzu LC-MS 2020 spectrometer by using electrospray ionization (ESI). Purification by flash column chromatography and prep-HPLC was performed using a Büchi Pure-Chromatography-System and Büchi FlashPure columns. IUPAC names of all compounds are provided and were determined using CS ChemDraw 19.1. All the code was written in Python and the machine learning the deep learning model was implemented using Python and TensorFlow 2 and Keras (Chollet, others, Keras (2015; https://keras.io); Abadi et al., TensorFlow: large-scale machine learning on heterogeneous systems (2015; https://www.tensorflow.org/)). All confusion matrices were plotted using Scikit-learn with matplotlib integrated (Pedregosa et al., Scikit-learn: machine learning in python. J Mach Learn Res. 12, 2825-2830 (2011); Hunter, Matplotlib: A 2D graphics environment. Comput Sci Eng. 9, 99-104 (2007)). The statistical plots were generated using the Seaborn package with the included statistical methods (Waskom, Seaborn: statistical data visualization. J. Open Source Softw. 6, 3021 (2021)).

### 3,3'-(Dimethylsilanediyl)bis(N,N-dimethylaniline) (S1)

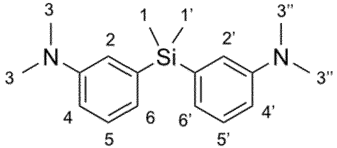

3-Bromo-*N*,*N*-dimethylaniline (5 g, 25 mmol) was dissolved in dry THF (60 ml) and the solution was cooled to -78 °C. *n*-Butyllithium (1.6 M, 17.2 mL, 27.5 mmol) was added dropwise and the mixture was stirred at -78 °C for 2 h. Dichlorodimethylsilane (1.52 mL, 12.5 mmol) was added dropwise and the mixture was stirred for 2 h at 25 °C. Brine (40 mL) and H₂O (10 mL) were added and the mixture was extracted three times with EtOAc. The combined organic phases were dried over MgSO₄ and concentrated onto Celite. The crude was purified by flash column chromatography (SiO₂; hexane to hexane/EtOAc 9:1) to give the product as a light-yellow oil (3.6 g, 50%).

¹H NMR (400 MHz, CDCl₃) δ = 7.31-7.19 (m, 2H, H5), 6.97-6.90 (m, 4H, H4, H2), 6.78 (dd, *J* = 8.3, 2.8, 2H, H6), 2.94 (s, 12H, H3), 0.55 (s, 6H, H1) ppm.

¹³C NMR (101 MHz, CDCl₃) δ = 154.75, 141.11, 131.49, 125.30, 120.50, 116.20, 42.84, 0.84 ppm.

### 3,3'-(Dimethylsilanediyl)bis(4-bromo-N,N-dimethylaniline) (S2)

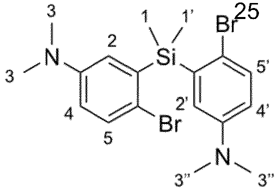

3,3'-(Dimethylsilanediyl)bis(*N*,*N*-dimethylaniline **S1** (3.00 g, 0.01 mol) was dissolved in CH₃CN (75 mL) in a flame-dried flask. The solution was cooled to 0 °C and *N*-bromosuccinimide (3.75 g, 0.021 mmol) was added in small portions. After complete addition, the solution was stirred for 1.5 h at 25 °C then sat. NaHCO₃ was added to neutralize the solution. The organic phase was washed with water. The combined aqueous phases were extracted twice with CH₂Cl₂. All CH₂Cl₂ fractions were combined dried over MgSO₄ and concentrated under reduced pressure. The residue was purified by flash column chromatography. (SiO₂; hexane: EtOAc 95:2) yielding a white product (2.69 g, 59%).

¹H NMR (400 MHz, CDCl₃) δ = 7.36 (d, *J* = 8.8, 2H, H5), 6.85 (d, *J* = 3.2, 2H H2), 6.61 (dd, *J* = 8.7, 3.2, 2H, H4), 2.89 (s, 12H, H3), 0.77 (s, 6H, H1) ppm.

¹³C NMR (101 MHz, CDCl₃) δ = 149.02, 138.86, 133.10, 121.92, 116.94, 115.40, 40.70, 27.05, -0.51, -0.78, -1.06 ppm.

### 5'-Bromo-N3,N3,N7,N7,5,5-hexamethyl-3'H,5H-spiro[dibenzo[b,e]siline-10,1'-isobenzofuran]-3,7-diamine (S3)

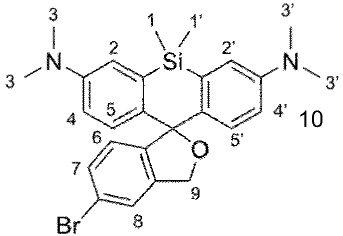

Compound **S2** (1 g, 2.19 mmol) in dry THF (10 mL) was added to magnesium turnings (213 mg, 8.77 mmol) in dry THF (3mL) at 60 °C followed by addition of 1,2-dibromoethane (40.6 µL, 470 µmol). The mixture was stirred at 25 °C for 3 h and the solution slowly turned yellow. The Grignard reagent was transferred into an empty flame-dried Schlenk flask and a solution of 5-bromophthalide (170 mg, 783 µmol) in dry THF (8 mL) was added at 60 °C. Upon addition, the reaction turned from deep yellow to dark brown. The mixture was stirred at the same temperature for 12 h. Aqueous HBr (3.8 mL, 47%) was added and the solution turned orange red and CH₂Cl₂ was added. NaHCO₃ was added very carefully until the color moved to the CH₂Cl₂ phase. The aqueous phase was extracted three more times with CH₂Cl₂. The organic phase was dried over Na₂SO₄, concentrated under reduced pressure and loaded onto Celite. Purification by reverse phase column chromatography (SiO₂-C₁₈; CH₃CN / H₂O + 0.1 TFA 5:95 to CH₃CN / H₂O + 0.1 TFA 95:5) gave the compound **S3** as the blue trifluoroacetate salt (302 mg, 45%).

¹H NMR (400 MHz, MeOD) δ = 7.90 (s, 1H, H8), 7.63 (d, *J* = 8.0, 1H, H7), 7.35 (d, *J* = 2.8, 2H, H2), 7.11-7.04 (m, 3H, H5, H6), 6.79 (dd, *J* = 9.7, 2.9, 2H, H4), 4.28 (s, 2H, H9), 3.35 (s, 12H, H3), 0.60 (d, *J* = 2.6, 6H, H1) ppm.

¹³C NMR (101 MHz, MeOD) δ = 167.63, 159.17, 158.76, 155.81, 149.41, 143.39, 142.14, 137.21, 132.07, 131.11, 131.05, 128.35, 124.14, 122.30, 120.28, 117.46, 115.33, 114.63, 111.80, 66.97, 61.74, 40.94, 27.20, -1.10, -1.34 ppm.

HRMS (ESI/QTOF) calcd. for [C₂₆H₃₀BrN₂OSi]⁺: 493.1305; found 493.1305.

### 5'-Iodo-N3,N3,N7,N7,5,5-hexamethyl-3'H,5H-spiro[dibenzo[b,e]siline-10,1'-isobenzofuran]-3,7-diamine (S4)

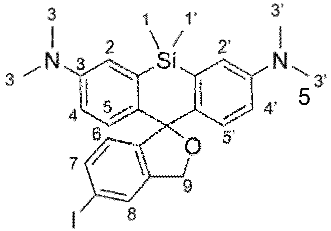

A flame dried microwave vial was charged with compound **S3** (49.5 mg, 100 µmol), Cul (9.52 mg, 50 µmol), Nal (300 mg, 2.00 mmol), and briefly evacuated and backfilled with N₂. Racemic *trans*-(1R,2R)-*N,N'*-dimethyl-cyclohexane-1,2-diamine (1.42 mg, 10 µmol), and dioxane (0.5 mL) were added. The microwave vial was sealed and the mixture was stirred at 110 °C for 29 h. The resulting suspension was cooled to reach 25 °C, diluted with 25% aq. NH₃, poured into water, and extracted with CH₂Cl₂. The combined organic phases were dried over Na₂SO₄ and concentrated under reduced pressure. The residue was purified by flash chromatography (SiO₂; hexane + 1% NH₃/ EtOAc + 1% NH₃ to 9:1 hexane + 1% NH₃ EtOAc + 1% NH₃) to provide product **S4** as a white solid (190 mg, 77%).

¹H NMR (400 MHz, CDCl₃) δ = 7.64 (s, 1H, H8), 7.53 (d, *J* = 8.0, 1H, H7), 7.24-7.14 (m, 2H, H2), 7.03 (d, *J* = 8.8, 2H, H5), 6.83 (dt, *J* = 8.8, 5.2, 2H, H4), 6.77 (s, 1H, H6), 5.08 (s, 2H, H9), 2.98 (s, 12H, H3), 0.56 (s, 3H, H1 or H1'), 0.49 (s, 3H, H1 or H1') ppm.

¹³C NMR (101 MHz, (CDCl₃) δ = 141.12, 136.61, 120.08, 118.10, 116.43, 114.68, 92.21, 0.38, -0.99 ppm.

HRMS (ESI/QTOF) calcd. for [C₂₆H₃₀IN₂OSi]⁺: 541.1167; found 541.1167.

### N-(((3H-Fluoren-8-yl)methoxy)carbonyl)-S-(3,7-bis(dimethylamino)-5,5-dimethyl-3'H,5H-spiro[dibenzo[b,e]siline-10,1'-isobenzofuran]-5'-yl)-L-cysteine (S5)

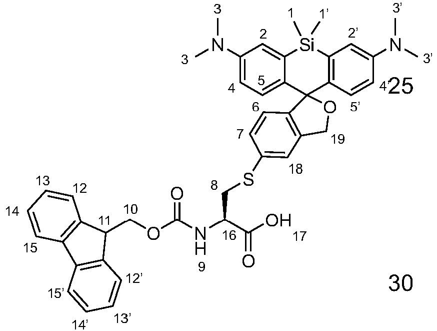

Xanthphos Pd G3 (15.8 mg, 16.7 µmol), Fmoc-L-Cys-OH (105 mg, 305 µmol) and compound **S4** (150 mg, 278 µmol) were dissolved in THF (1.4 mL) in a flame-dried Schlenk flask. The mixture was stirred at 25 °C for 2-3 min, then NEt₃ (65.6 µL, 472 µmol) was added, the Schlenk tube was capped with a rubber septum, evacuated and backfilled with argon. The solution was stirred at 25 °C for 30 min. The solvent was removed under reduced pressure. The residue was loaded onto Celite and purified by flash chromatography (SiO₂; CH₂Cl₂ to CH₂Cl₂/CH₃OH 95:5) to yield a blue/green powder (200 mg, 95%).

¹H NMR (400 MHz, THF) δ = 7.77 (d, *J* = 7.5, 2H, H15), 7.66 (t, *J* = 8.2, 2H, H12), 7.40 (s, 1H, H18), 7.34 (t, *J* = 7.4, 2H, H14), 7.25 (dd, *J* = 8.7, 5.4, 3H, H7 and H12), 7.03 (dd, *J* = 8.7, 3.0, 3H, H5 and H9), 6.96 (t, *J* = 3.1, 2H, H2), 6.86 (dd, *J* = 8.0, 4.4, 1H, H6), 6.64-6.55 (m, 2H, H4), 5.21 (s, 2H, H19), 4.49 (td, *J* = 8.0, 4.6, 1H, H16), 4.28 (dd, *J* = 7.3, 2.1, 1H, H10), 4.23 (s, 1H, H11), 3.49 (dd, *J* = 13.5, 4.8, 1H, H8), 3.25 (dd, *J* = 13.5, 7.9, 1H, H8'), 2.89 (d, *J* = 4.3, 12H, H3), 0.58 (s, 3H, H1), 0.46 (s, 3H, H1') ppm.

¹³C NMR (101 MHz, THF) δ = 172.37, 160.18, 159.90, 157.01, 149.88, 148.42, 145.40, 145.37, 142.39, 135.89, 129.70, 128.49, 127.93, 126.26, 126.24, 125.18, 123.93, 120.76, 118.43, 115.44, 74.24, 55.05, 54.84, 54.74, 48.38, 46.81, 42.03, 38.27, 37.32, 0.51, -0.64 ppm.

HRMS (ESI/QTOF) calcd. for [C₄₄H₄₆N₃O₅SSi]⁺: 756.2922; found 756.2913.

### N-(((9H-fluoren-9-yl)methoxy)carbonyl)-O-((benzyloxy)(hydroxy)phosphoryl)-L-serine (S6)

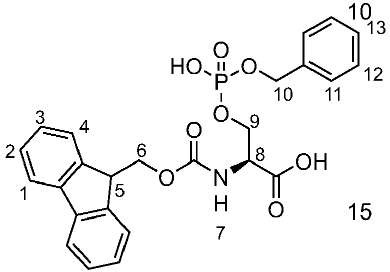

Water from Fmoc-*L*-Ser-OH·H₂O was azeotropically distilled from THF (4 mL g⁻¹) on the rotary evaporator and dried overnight. PCl₃ (379 µL, 4.34 mmol) was dissolved in THF (8.0 mL) and cooled to 0 °C. Benzyl alcohol (518 µL, 5 mmol) was added while keeping the internal temperature below 5 °C. The solution was stirred for 10 minutes at 0-5 °C. The reaction was further cooled to -5 °C then 2,6-lutidine (1.17 mL, 10 mmol) was added to the flask keeping the reaction at -5 to 5 °C, forming a thick slurry. 2,6-Lutidine (389 µL, 3.34 mmol) was added to a solution of Fmoc-Ser-OH (1.15 g, 3.34 mmol) in THF (4.0 mL) in a separate flask. This solution was added to the reaction at a rate that kept the mixture at -5 to 5 °C. Upon reaction completion, H₂O (3.6 mL) was added to the flask, maintaining the temperature below 10 °C followed by addition of NaBr (789 mg, 7.67 mmol) at 0 °C. A 20% w/w aqueous solution of NaBrO₃ (252 mg, 1.67 mmol) was added at 0-5 °C. After the addition, the mixture was warmed to 25 °C. Upon reaction completion, an aqueous solution of Na₂S₂O₅ (20% in H₂O) (1.0 mL) was added to the flask in one portion. 2-CH₃-THF was added, and the layers were shaken and separated. The organic layer was washed with brine, dried with Na₂SO₄, and concentrated under reduced pressure. The crude oil was diluted with 2-CH₃-THF (7 mL g⁻¹), stirring at ambient temperature for 16 h, yielded a white precipitate, which was filtered of and washed with cold 2-CH₃-THF. The product was obtained as a white solid (700 mg, 42%).

¹H NMR (400 MHz, DMSO-*d*₆) δ = 7.89 (d, *J* = 7.6 Hz, 2H, H1), 7.84 (d, *J* = 8.3 Hz, 1H, H7), 7.73 (d, *J =* 7.5 Hz, 2H, H4), 7.45-7.27 (m, 9H, H2, H3, H11, H12 and H13), 4.93 (d, *J* = 7.1 Hz, 2H, H10), 4.36-4.08 (m, 6H, H5, H6, H8, H9).

¹³C NMR (101 MHz, DMSO-*d*₆) δ = 170.75, 155.98, 143.76, 143.74, 140.68, 136.77, 136.69, 128.36, 128.03, 127.64, 127.60, 127.08, 125.30, 120.10, 67.52, 67.47, 65.92, 65.28, 65.23, 54.40, 54.32, 46.55. ³¹P NMR (162 MHz, DMSO) δ = -1.49.

HRMS (ESI): *m*/*z* calcd. [C₂₅H₂₄NO₈P]⁺: 498.1312; Found 498.1327.

### Section 2. Peptide synthesis.

Peptides **C1-C4** and **P1-P3** were prepared by manual SPPS, whereas peptides **E1-E3** were prepared on an automated Tribute^{™} UV-IR peptide synthesizer (Gyros Protein Technologies AB) with manual coupling of the HMSiR labeled cysteine at an intermediate step. All peptides were synthesized on a 0.05 mmol scale.

**Manual peptide synthesis:** After every reaction and swelling step, the resin was washed three times with each CH₂Cl₂ and DMF (4 mL). The swelling and all the reaction steps were shaken at room temperature using a heating/cooling drybath (Thermo Scientific). Peptides were synthesized on 100 mg pre-loaded Wang-resins (Bachem, 0.5 - 0.8 mmol g⁻¹, 1 equiv.), which were swollen at least 3 h in 4:1 CH₂Cl₂/ DMF. Standard, manual Fmoc-SPPS protocols were used for synthesis. Briefly, deprotection was performed with 20% piperidine in DMF for 20 min and coupling with *O*-(1H-Benzotriazol-1-yl)-*N,N,N',N'-*tetramethyluroniumhexafluorophosphat (HBTU) (4 equiv.), DIPEA (8 equiv.) and Fmoc-Xaa-OH (4 equiv.) in DMF for 1 h. Fmoc-Cys(HMSiR)-OH **S5** was coupled with 1-hydroxbenzotriazole (HOBt) (2.5 equiv.), N-Ethyl-N'-(3-dimethylaminopropyl) carbodiimide hydrochloride (EDC HCl) (2.5 equiv.), Fmoc-Cys(HMSiR)-OH **S5** (2.5 equiv.). The solids for Fmoc-Cys(HMSiR)-OH **S5** coupling were premixed in 3 mL DMF: THF 1:1 for 5 min, added to the deprotected resin and left shaking for 3 h. For the coupling phosphorylated amino acid **S6** the equivalents of the amino acid **S6,** coupling reagent, HBTU, and DIPEA were increased by one to 5 and 9 equivalents respectively. The final coupling was done by pre-mixing pentynoic acid (10 equiv.) with EDC HCl (10 equiv.) in 2 mL of 1:1 CH₂Cl₂/ DMF after 2 min the mixture was added to the resin for 2 h.

Cleavage from the resin and concurrent global deprotection was carried out with a mixture of trifluoroacetic acid (TFA), water and triisopropylsilane (TIPS) (90:5:5) for 3 h. The crude products were purified by preparative HPLC (A: H₂O + 0.1% TFA, B: CH₃CN; 1. 0-10 min, 20%-50% B; 2. 10-20 min, 50% B; 3. 20-24 min, 50-20% B). The obtained peptides were characterized by LC-MS, ESI-HRMS and ESI-MS/MS. They were stored as 1 mM stock solutions in dimethyl sulfoxide and in lyophilized form.

**Automated peptide synthesis:**The peptides **E1-E3** were synthesized on a Tribute UV-IR (Gyros Protein Technologies) peptide synthesizer applying the manufacturer's standard cycles. The couplings were conducted with Fmoc-Xaa-OH (5 equiv.), O-(1H-6-chlorobenzotriazole-1-yl)-1,1,3,3-tetramethyluronium (HCTU) (4.75 equiv.) and DIPEA (7 equiv.) in DMF. The Fmoc-deprotection was done with 20% piperidine in DMF. The coupling of the Fmoc-Cys(HMSiR)-OH **S5** amino acid was conducted manually with Fmoc-Cys(HMSiR)-OH **S5** (2.5 equiv.), HOBt (2.5 equiv.), EDC HCl (2.5 equiv.). The solids were premixed in 3 mL DMF: THF 1:1 for 5 min, added it to the deprotected resin and left shaking for 3 h. The final coupling was done manually by pre-mixing pentynoic acid (10 equiv.) with EDC HCl (10 equiv.) in 2 mL of 1:1 CH₂Cl₂/ DMF after 2 min the mixture was added to the resin for 2 h. After the manual steps the resin was washed with each CH₂Cl₂ and DMF (4 mL).

The peptides were cleaved from the resin by addition of 3 mL of Reagent K ((TFA, phenol, water, thioanisole, 1,2-ethanedithiol in ratio; 82.5:5:5:5:2.5) mixing for 3 h. After concentration of the cleavage mixture cooled tert-butyl methyl ether (MTBE) was added to the residue. The precipitate was centrifuged for 15 min at 4000 rpm, the supernatant was removed, the pellet was resuspended in MTBE and centrifuged once more. The crude products were purified by HPLC. The obtained peptides were characterized by ESI-HRMS and ESI-MS/MS. They were stored as 1 mM stock solutions in dimethyl sulfoxide and in lyophilized form.

### Section 3. Preparation of glass slides.

The fluorophore-peptide conjugates were covalently bound to glass slides that were extensively cleaned, followed by passivation using a mixture of heterobifunctional polyethylene glycol derivatives (PEGs), adapted from published procedures (Jain et al., Nat. Protoc. 7, 445-452 (2012)). For passivation a mixture of PEGs that are non-reactive (methoxy-PEG₅₀₀₀-NHS) and reactive (azide-PEG₅₀₀₀-NHS) in a "click" reaction. This mixture allowed for sparse single-molecule signals on the glass surface.

**Surface passivation:** The glass containers and Erlenmeyer dedicated to the aminosilation step were sonicated first with 1 M KOH, rinsed with ultrapure water five times, sonicated for 20 min in CH₃OH and dried under a stream of nitrogen. The coverslips (always in pairs) were placed into the cleaning containers and rinsed with ultrapure water. A 10% Alconox solution was added and the slides were sonicated for 20 minutes. The slides were rinsed with tap water until no bubbles were apparent, three times with distilled water, twice with ultrapure water and sonicated for 5 min in ultrapure water. The container was rinsed with acetone once and refilled with acetone and sonicated for 20 min. After a last rinse with acetone the coverslips were dried under a stream of N₂ and placed in the active area of a UV/ozone cleaner (Jelight Company Inc., UVO-Cleaner Model No. 256-220) and treated for 10 minutes followed by 5 min of resting time. The cleaned and activated coverslips were transferred into the containers dedicated to the aminosilation step. The aminosilation solution was freshly prepared in the cleaned Erlenmeyer by adding 2 mL of AEAPTES to 98 mL of acetone (HPLC grade) and mixing with a dedicated glass rod. The premixed solution was added to the slides, and they were placed in a dark, well-leveled place. After 10 min the containers were sonicated for 1 min and then put back in the dark for another 10 min. The aminosilation solution was discarded and the slides were rinsed with acetone once and three times with ultrapure water before drying them with N₂ and placing them in the PEGylation container (a pipette tip box with tips on the edges of the cover slips, to prevent them from touching the box with the clean surface).

For PEGylation the buffer and the solution were freshly prepared right before application. For five pairs of coverslips, 1 mg azide-PEG5000-NHS and 16 mg methoxy-PEG5000-NHS were added to an Eppendorf tube and dissolved in 320 µL PEGylation buffer (1 mM sodium bicarbonate solution), this solution was mixed gently and centrifuged for 1 min at 10 000 rpm. 70 µL of PEGylation solution was added onto the functionalized side of half of the coverslips, then a second coverslip was gently placed face down on top of the solution without introducing any bubbles. Water was added in the bottom of the pipette box and the slides were kept closed in a dark, well-leveled place for 3 h. The coverslips were taken apart, rinsed with ultrapure water and CH₃OH from a squirt bottle and dried with compressed nitrogen.

For the second PEGylation step, 7 mL of a 250 mM MSPEG4 solution in anhydrous DMF was added to 63 mL of PEG buffered (prepared before) and added in the same manner to a pair of coverslips. The slide-sandwiches were incubated overnight. The slides were taken apart and rinsed with CH₃OH and ultrapure water, and dried under a stream of N₂.

**Click functionalization standard procedure:** The coverslips were placed back into the pipette tip boxes with the passivated surface face up. For the measurement of three different peptides in one experiment, a master-mix with the common components was prepared: 45 mL of a 100 mM THPTA (10 mM, final concentration) were combined with 9 mL of a 100 mM copper (II) sulfate solution (2 mM, final concentration). The reagents were diluted with 351 mL of a 3:1 water/glycerol mixture.

From this diluted solution, 135 mL were transferred to a new Eppendorf tube, then 0.15 mL of the corresponding 1 mM peptide-dye conjugate stock solution was added, followed by 15 mL of 1 M sodium ascorbate. The reaction mix was vortexed thoroughly before applying 70 mL of the final reaction solution to the reactive surface of a passivated glass slide. A second glass coverslip is gently placed on top, with the passivated side facing down, i.e. towards the reaction solution, without introducing any bubbles. After 2 h the slides were slid apart and rinsed with ultrapure water, 25 mM enediaminetetraacetic acid (EDTA) solution, ultrapure water, acetone, EtOAc, CH₃OH, and finally again with ultrapure water. The functionalized coverslips were dried dry under a stream of N₂, placed in a KimWipe^{™} lined container and transported to the microscope for imaging.

### Section 4. Fluorescence imaging.

Single-molecule movies were acquired either on a Nikon N-STORM microscope (Nikon, UK Ltd.) with an SR Apochromat TIRF 100x 1.49 N. A. oil immersion objective lens. A piezo-electronic focus-lock system (perfect focus system) was used to prevent axial drift during data acquisition. The illumination power of the diode-pumped solid-state laser was measured at the tip of the optical fiber as 638 nm, 110 mW. The emission was passed through a filter with a band pass window at 683-783 nm. The fluorescence was detected with an EMCCD camera Andor iXon Ultra 888. The microscope was operated using the NIS Elements (Nikon) software. For imaging, the slides were fixed in a cell chamber and rinsed twice with 1 mL of imaging buffer (10 mM sodium phosphate buffer at pH = 7.4), 1 mL of imaging buffer was added for imaging. The coverslip was then imaged in TIRF mode in the 641 or 638 nm channel at 80% or 100 % laser power respectively with an exposure time of 30 ms. The conditions were kept constant for all acquisitions. A field of view was imaged for 6000 frames. For all peptides, single-molecule measurements were carried out on three different days and at least two different coverslips per day, and all these signals were mixed to create the dataset for further analysis.

### Section 5. Data analysis.

To perform the classification analysis, we first localized the single-molecule signals, extracted the numeric intensity values of the signal over all frames followed by data normalization, filtering to remove noise signals as described below. For classical machine learning, we continued with feature extraction, standardization, and model training. For deep learning the standardized and normalized traces were used directly as input for the model. All deterministic machine learning analysis was conducted on a desktop computer with an AMD Ryzen 9 3900X 12-Core, 3800 MHz Processor, a GeForce RTX 2070 Super graphics card and 128 GB of physical memory. The deep learning model was trained using the GPU. The Monte Carlo dropout models were trained on the ScienceCloud and ScienceCluster service infrastructure provided by S3IT (www.s3it.uzh.ch), the Service and Support for Science IT team at the University of Zurich.

**Fluorescence trace extraction:** In each frame all the single molecules were localized using the freely available single-molecule localization package Picasso (Schnitzbauer et al., Nat Protoc. 12, 1198-1228 (2017) with the settings listed in table 1. The obtained framewise localization were combined using the Picasso postprocess module. All the localization within a two-pixel distance from the first appearance of a localization were combined as one particle and the final location of the particle was calculated from the averaged x- and y-coordination of the combined localization that were detected. These mean locations were exported for all the particles and further used to obtain the fluorescence intensity trace of each particle with custom written code was defined as the center of a box with a side length of five pixels. The pixel intensities within this box were summed up and recorded as the particle intensity for the respective frame, resulting in an intensity trace over all the frames. All boxes that overlapped in more than two pixels were not considered, as well as boxes that overlapped with the edge of the field of view. All traces were recorded and saved in reference to their peptide of origin **(C1-C4, P1-P3,** or **E1-E3).**

**Fluorescence trace preprocessing and filtering:** The traces obtained from the movies needed to be standardized to remove potential influences from the different technical replicates (different coverslips and different days). Therefore, we used the last 500 frames, which were bleached in most traces, to estimate the mean value of the background of the acquisition, which were subtracted from each frame in the trace. Furthermore, we standardized the fluorescence intensity values by either calculating the z-score for each value in the traces or by producing the min-max normalized traces. The z-scored traces were used to determine the peaks in each trace using the function find_peaks, contained in the SciPy python library. As a threshold for peak detection, we set a minimum intensity of eight standard deviations (8s) from the background. These peaks were the basis for filtering the traces to remove potential traces stemming from background noise or impurities in the experiment. The applied filtering criteria are listed in table 2.

**Feature extraction for classical machine learning:** The pre-filtered traces were used to extract features manually for each trace. The peaks were again determined but with a lower threshold of five standard deviations (5s) to avoid missing any peak of lower intensity that might occur due to a very short-lived fluorescent signal. From the peak list and their properties obtained from the find_peaks function, we determined the features in table 3.

**Feature-based classification:** The features (table 3) were further standardized to avoid features covering a large numeric range from dominating the classification over the features in a smaller numeric range. The features were used to create a random training and validation set. The classical ML models were implemented using Scikit-learn (Pedregosa et al., ibid.). We tested the models: AdaBoost, multilayer perceptron, K-nearest neighbors, support vector machine with a radial basis function, decision tree, and random forest with the default parameters.

**Data augmentation for deep learning:** To increase the amount of data for improved learning of model training, we generated traces with a mirror-image peak region. We defined the peak region between the left-bound of the first and the right-bound of the last detected peak. This partial sequence was inverted in place, to obtain the trace with a mirrored peak-region.

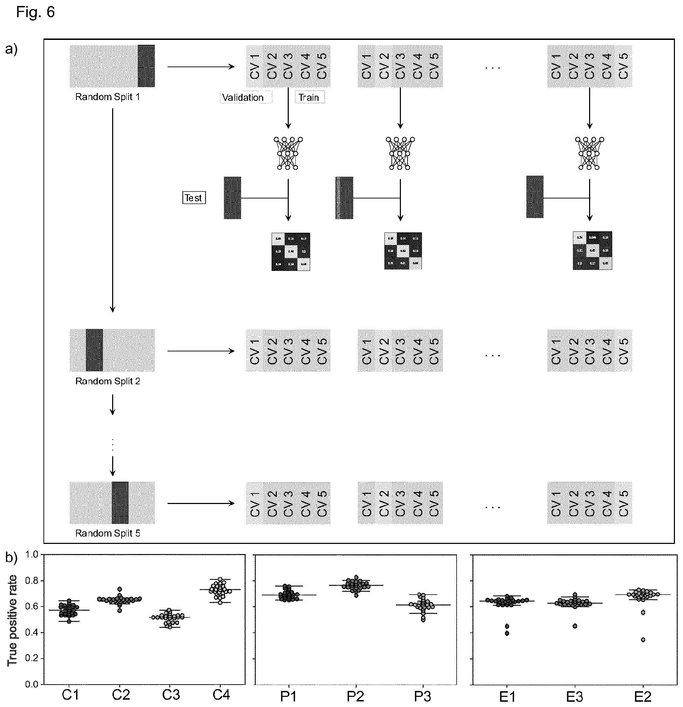

**Deep learning settings and classification using the deterministic approach 1D-CNN-GRU:** The deep learning model (table 4) was implemented using Python and TensorFlow 2.7 with the Keras API. The hyperparameters used in the model are described in table 4. We used Adam (Kingma and Ba, Adam: a method for stochastic optimization. ArXiv14126980 Cs (2017) (available at http://arxiv.org/abs/1412.6980)) optimizer, with the default settings, with a mini-batch size of 32 for the compound peptides set **C1-C4** and **P1-P3** and size of 128 for the set **E1-E3.** The traces obtained after preprocessing, filtering and data augmentation were used for the deep learning approach. The z-scored and min-max scaled traces were used as separate features for each trace and directly used as input vectors to the model (table 4). To prevent overfitting and stop the training at the best performance on the validation set, we used early stopping with patience of 10 epochs. For a more general sense of model behavior and to avoid potential outlier results, the model was trained using a nested cross validation approach (Fig. 6). We report the mean value of all the folds in the confusion matrices (Figs. 2 and 3) and the variation of the true positive rate along with the mean overall accuracies and standard deviations of the model evaluation on a held-out test set that was not used in model training. We performed all the modeling experiments using a seed of 42.

**Monte Carlo dropout implementation, 1D-CNN-GRU-MCD:** The traces were preprocessed as in the deterministic approach and the same input was used for the probabilistic model 1D-CNN-GRU-MCD. To make the model probabilistic, custom Dropout and GRU layers were used, which use Monte Carlo dropout at inference time. As Monte Carlo dropout was used in the GRU layer itself, the input Dropout layer was removed. For evaluation, 100 predictions were calculated for each trace in the validation/test set yielding 100 potentially different predictions depending on the dropped nodes by the dropout mechanism. The mean model output over the 100 predictions was calculated per class used to predict the label.

**Definition of the classification certainty factor (CF) and its application as a certainty threshold:** The uncertainty quantification was calculated as the Wasserstein distance (using the SciPy implementation of the Wasserstein distance) (Virtanen et al., SciPy 1.0: fundamental algorithms for scientific computing in Python. Nat Methods. 17, 261-272 (2020)) between the class with the highest mean probability over the 100 predictions and the closest other class in the classification problem. Therefore, the prediction with the highest mean was determined and the Wasserstein distance for the other two or three classes in the problem were calculated, the minimal distance was determined and stored as the CF value. Importantly, the CF value does not depend on whether the class with the highest probability is actually correct. These CF values were used to filter uncertain traces if their minimal distance was larger than the chosen CF threshold. All traces, that were not filtered out based on the CF threshold were used to calculate the accuracy score.

### Tables

**Table 1: Settings applied in the single-molecule localization software Picasso (Schnitzbauer et al., Nat Protoc. 12, 1198-1228 (2017). MLE = maximum likelihood estimation.)**

| **Parameter** | **Value** |
|---|---|
| Box side length | 5 px |
| Fit method | MLE |
| Gradient | 7000 |
| Baseline | 100 |
| Sensitivity | 15.6 |
| Gain | 300 |
| Quantum yield | 0.93 |

**Table 2. Filter parameters applied in custom code to remove potential noisy traces.**

| **Parameter** | **Value** |
|---|---|
| Threshold for peaks (in number of standard deviations) | 8 |
| Minimal peak width (in frames) | 1 |
| Minimal peak number | 10 |
| Difference in #frames between 1^{st} and 2^{nd} peak | MLE |
| Last frame for a first peak to occur | 100 |
| Fourier transform maximal intensity | <1000 |

**Table 3. A list of features extracted for the classical machine learning approach.**

| **Feature** |
|---|
| Number of peaks |
| First peak |
| Last peak |
| Blinking time (...) |
| Maximal peak height |
| Mean peak height |
| Std peak height |
| Maximal peak width |
| Mean peak width |
| Std peak width |
| Maximal approximate peak area |
| Minimal approximate peak area |
| Mean approximate peak area |
| Std approximate peak area |
| Tempo |
| Last frame for a first peak to occur |

**Table 4. The model architecture in detail with all the parameters that were set manually.**

| **Layer** | **Layer settings** | **Param #** |
|---|---|---|
| Input | input shape=(None, 6000, 2) | |
| Convolutional layer 1D 1 | number of filters=64, kernel size=9, stride size=2, activation="relu" | 1216 |
| Batch Normalization | - | 256 |
| Dropout | dropout rate=0.3 | 0 |
| Convolutional layer 1D 2 | number of filters=64, kernel size=3, stride size=2, activation="relu" | 12352 |
| Batch Normalization | - | 256 |
| Dropout | dropout rate=0.5 | 0 |
| Convolutional layer 1D 3 | number of filters=64, kernel size=3, stride size=2, activation="relu" | 12352 |
| Batch Normalization | - | 256 |
| Dropout | dropout rate=0.3 | 0 |
| GRU layer | units=128 | 74496 |
| GRU layer | units=256 | 296448 |
| Dense layer | units=number of classes, activation="softmax" | 771 (for 3 classes) |

Parameters that are not mentioned were used in their default value as set in TensorFlow 2.7.0.

**Table 5. The model architecture including Monte Carlo dropout in detail with all the parameters that were set manually. Parameters that are not mentioned were used in their default values as set in TensorFlow 2.7.0.**

| **Layer** | **Layer settings** |
|---|---|
| **Input** | input shape=(None, 6000, 2) |
| **Convolutional layer 1D 1** | number of filters=64, kernel size=9, stride size=2, activation="relu" |
| **Batch Normalization** | - |
| **Dropout** | dropout rate=0.1 |
| **Convolutional layer 1D 2** | number of filters=64, kernel size=3, stride size=2, activation="relu" |
| **Batch Normalization** | - |
| **Dropout** | dropout rate=0.3 |
| **Convolutional layer 1D 3** | number of filters=64, kernel size=3, stride size=2, activation="relu" |
| **Batch Normalization** | - |
| **GRU layer** | units=128 with Dropout=0.05 |
| **GRU layer** | units=256 with Dropout=0.05 |
| **Dense layer** | units=number of classes, activation="softmax" |

### Description of the Figures

- Fig. 1: **Principle of peptide fingerprinting by blinkognition.** (A) Chemical structure of the spontaneously blinking fluorophore HMSiR (18). (B) The blinking pattern (intensity over time) of the fluorophore should be different when it is attached to different peptides. (C) Peptide-fluorophore conjugates are attached via click chemistry to a PEGylated glass surface and imaged using TIRF microscopy. (D) Time-lapse imaging produces blinking time traces that can be used to extract features for ML-based identification of peptide sequences.
- Fig. 2: **Single-molecule classification results for peptide set C1-C4. (A)** Structures of peptides **C1-C4** indicating the blinking fluorophore attached to cysteine (C) residue and the *N*-terminal alkyne for surface conjugation (SEQ ID NO: 1-4). **(B)** Confusion matrices from classical ML models: support vector machine with radial basis function (SVM-RBF), random forest, multilayer perceptron, or AdaBoost. **(C)** Simplified scheme of the 1D-CNN-GRU model architecture used for single-molecule classification. **(D)** Confusion matrix displaying results using the 1D-CNN-GRU classifier. **(E)** Results obtained when the identities of the traces are scrambled during training.
- Fig. 3: **Quantification of classification certainty and increased accuracy for the C1-C4 peptide set. (A)** Schematic representation of certainty quantification using the Monte Carlo dropout approach. Each trace in the test set is evaluated on n = 100 partial models, resulting in a distribution of classification outputs. **(B)** Classification accuracy obtained for signals with a CF value above the given threshold. The color of the dots indicates the percentage of discarded traces. The dashed red line indicates the chosen threshold (0.7) for this peptide set. **(C)** Confusion matrix for the classification of peptides **C1-C4** using a CF value of 0.7.
- Fig. 4: **Single-molecule classification results for peptide sets P1-P3 and E1-E3.** (A) Structures of peptides **P1-P3** (SEQ ID NO: 5-7). The chemical structures of serine (S) and phosphoserine are displayed in the box. (B) Confusion matrix displaying the results for the classification of peptides **P1-P3.** (C) Structures of peptides **E1-E3** (SEQ ID NO: 8-10). The chemical structures of L-isoleucine (I) and L-valine (V), as well as their epimers D-*allo*-isoleucine (i) and D-valine (v) are displayed in the box. (D) Confusion matrix displaying the results for the classification of peptides **E1-E3.**
- Fig. 5:: General overview of the synthesis of the HMSiR-Fmoc-cysteine building block **S5** that was used in SPPS to synthesize the model peptides with site-specific labelling.
- Fig. 6: Nested cross validation. a) Evaluation of the model (table 4) on a dataset. The fluorescence intensity traces are split into a train/validation set and a test set. The test set is kept aside while a model is trained using the training/validation set in a five-fold cross validation (CV) approach. Each model resulting from a CV training is evaluated on the test set that split off initially. This whole process was repeated five times to reduce the influence of the initial split into a train/validation set and a test set on the evaluation. b) Plots of the true positive values obtained for all the 25 models trained in the five random splits with five-fold CV. The middle line represents the median of all the values, while the whiskers represent the quartiles, without outliers as determined in the Seaborn implementation (5). For the epimers set we can observe outliers in the evaluation results, resulting from models that did not converge, most likely due to exploding gradients during training.

### Cited prior art documents:

Blinking dyes: Lukinavicius et al., Nature Chemistry 6(8):663-664; Lardon, Wang, Johnsson et al., Systematic Tuning of Rhodamine Spirocyclization for Super- Resolution Microscopy, BioRxiv 2011; Tyson et al., ACS Cent Sci. 2021 Aug 25;7(8):1419-1426; Unoet al., Nat. Chem. 6, 681-689 (2014); Zheng et al., ACS Cent. Sci. 5, 1602-1613 (2019); Halabi et al., Nat. Commun. 10, 1232 (2019); Tachibana et al., Chem. Commun, 56, 13173-13176 (2020); Uno et al., Chem. Commun., 54,102-105 (2018).
Gal, Z. Ghahramani, Dropout as a Bayesian approximation: Representing model uncertainty in deep learning. Proc. 33rd Int. Conf. Mach. Learn. 48 (2016)
Cho et al., On the properties of neural machine translation: encoder-decoder approaches. arXiv:1409.1259v2 [cs.CL]
Zanon PRA, Yu F, Musacchio P, Lewald L, Zollo M, Krauskopf K, et al. Profiling the proteome-wide selectivity of diverse electrophiles. ChemRxiv. Cambridge: Cambridge Open Engage; 2021

## Claims

1. A method for identifying a variant of a biomolecule among a plurality of variants of the biomolecule, said method comprising the steps of
a. acquiring a fluorescence intermittency signal pattern for each variant of the plurality, wherein each of the variants is labelled with a fluorophore exhibiting spontaneous fluorescence intermittency upon excitation;
b. determining and associating a fluorescence intermittency signal characteristic for each of said variants;
c. providing a sample comprising a variant of the plurality, and labelling the variant in the sample with said fluorophore, and acquiring a fluorescence intermittency signal from the labelled variant;
d. determining a presence of a fluorescence intermittency signal characteristic as determined in step b. in the acquired fluorescence intermittency signals from the sample;
e. establishing the presence and optionally, the quantity and/or localisation, of a variant of the biomolecule in the sample based on the fluorescence intermittency signal characteristic determined from the fluorescence intermittency signal acquired from the sample and the fluorescence intermittency signal characteristic determined in step b.

2. The method according to claim 1, wherein the fluorophore is covalently linked to the biomolecule.

3. The method according to claim 1 or 2, wherein the biomolecule is a peptide.

4. The method according to claim 3, wherein the peptide is composed of proteinogenic amino acids.

5. The method according to any one of the previous claims, wherein the biomolecule comprises a thiol group, particularly wherein the biomolecule comprises a cysteine residue, and the fluorophore is covalently linked to the cysteine.

6. The method according to any one of the previous claims, wherein the fluorophore is **characterized by** blinking in the millisecond time scale.

7. The method according to any one of the previous claims, wherein the fluorophore is a silicon rhodamine, particularly a hydroxmethyl silicon rhodamine.

8. The method according to any one of the previous claims, wherein the biomolecule is a peptide, and said variants differ from the peptide with regard to their:
a. amino acid sequence,
b. posttranslational modification of their amino acid sequence, particularly with regard to a posttranslational modification selected from the group consisting of epimerization, deamidation, citrullination, phosphorylation, ribosylation, glycosylation, acetylation, methylation, hydroxylation;
particularly wherein the posttranslational modification is selected from the group consisting of epimerization, deamidation, and citrullination;
c. conformational changes of the biomolecule due to interactions with other biomolecules or its environment.

9. The method according to any one of the previous claims, wherein the biomolecule is anchored to a surface, particularly a solid surface, a cell surface or a vesicle surface.

## Patentansprüche

1. Ein Verfahren zur Identifizierung einer Variante eines Biomoleküls unter einer Vielzahl von Varianten des Biomoleküls, wobei das Verfahren die folgenden Schritte umfasst
a. erfassen eines Fluoreszenz-Intermittenz-Signalmusters für jede Variante der Vielzahl, wobei jede der Varianten mit einem Fluorophor markiert ist, das bei Anregung eine spontane Fluoreszenz-Intermittenz aufweist;
b. bestimmen und zuordnen eines für jede der genannten Varianten charakteristischen Floureszenz-Intermittenzsignals;
c. bereitstellen einer Probe, die eine Variante aus der Vielzahl enthält, und markieren der Variante in der Probe mit besagtem Fluorophor und erfassen eines Fluoreszenz-Intermittenzsignals der markierten Variante;
d. bestimmen eines Vorhandenseins eines charakteristischen Fluoreszenz-Intermittenzsignals, das in Schritt b) bestimmt wurde, in den von der Probe erfassten Fluoreszenz-Intermittenzsignalen;
e. feststellen des Vorhandenseins und optional der Menge und/oder der Lokalisierung einer Variante des Biomoleküls in der Probe basierend auf dem charakteristischen Fluoreszenz-Intermittenzsignal, das aus dem von der Probe erfassten Fluoreszenz-Intermittenzsignal und dem in Schritt b bestimmten charakteristischen Fluoreszenz-Intermittenzsignal ermittelt wurde.

2. Das Verfahren nach Anspruch 1, wobei das Fluorophor kovalent an das Biomolekül gebunden ist.

3. Das Verfahren nach Anspruch 1 oder 2, wobei das Biomolekül ein Peptid ist.

4. Das Verfahren nach Anspruch 3, wobei das Peptid aus proteinogenen Aminosäuren zusammengesetzt ist.

5. Das Verfahren nach einem der vorherigen Ansprüche, wobei das Biomolekül eine Thiolgruppe umfasst, insbesondere wobei das Biomolekül einen Cysteinrest umfasst, und das Fluorophor kovalent an das Cystein gebunden ist.

6. Das Verfahren nach einem der vorherigen Ansprüche, wobei das Fluorophor **dadurch gekennzeichnet ist, dass** es im Millisekundenbereich blinkt.

7. Das Verfahren nach einem der vorherigen Ansprüche, wobei das Fluorophor ein Silicium-Rhodamin ist, insbesondere ein Hydroxymethyl-Silicium-Rhodamin.

8. Das Verfahren nach einem der vorherigen Ansprüche, wobei das Biomolekül ein Peptid ist und sich die genannten Varianten vom Peptid hinsichtlich ihrer:
a. Aminosäuresequenz,
b. posttranslationalen Modifikation ihrer Aminosäuresequenz, insbesondere im Hinblick auf eine posttranslationale Modifikation, ausgewählt aus der Gruppe bestehend aus Epimerisierung, Deamidierung, Citrullinierung, Phosphorylierung, Ribosylierung, Glykosylierung, Acetylierung, Methylierung und Hydroxylierung;
insbesondere wobei die posttranslationale Modifikation aus der Gruppe bestehend aus Epimerisierung, Deamidierung und Citrullinierung ausgewählt ist;
c. Konformationsänderungen des Biomoleküls aufgrund von Wechselwirkungen mit anderen Biomolekülen oder seiner Umgebung
unterscheiden.

9. Das Verfahren nach einem der vorherigen Ansprüche, wobei das Biomolekül an einer Oberfläche, insbesondere einer festen Oberfläche, einer Zelloberfläche oder einer Vesikeloberfläche, verankert ist.

## Revendications

1. Procédé d'identification d'un variant d'une biomolécule parmi une pluralité de variants de la biomolécule, ledit procédé comprenant les étapes consistant à
a. acquérir un motif de signal d'intermittence de fluorescence pour chaque variant de la pluralité, dans lequel chacun des variants est marqué avec un fluorophore présentant une intermittence de fluorescence spontanée lors de l'excitation ;
b. déterminer et associer une caractéristique de signal d'intermittence de fluorescence pour chacun desdits variants ;
c. fournir un échantillon comprenant un variant de la pluralité, et marquer le variant dans l'échantillon avec ledit fluorophore, et acquérir un signal d'intermittence de fluorescence à partir du variant marqué ;
d. déterminer une présence d'une caractéristique de signal d'intermittence de fluorescence telle que déterminée à l'étape b. dans les signaux d'intermittence de fluorescence acquis à partir de l'échantillon ;
e. établir la présence et en option, la quantité et/ou localisation, d'un variant de la biomolécule dans l'échantillon en fonction de la caractéristique de signal d'intermittence de fluorescence déterminée à partir du signal d'intermittence de fluorescence acquis à partir de l'échantillon et de la caractéristique de signal d'intermittence de fluorescence déterminée à l'étape b.

2. Procédé selon la revendication 1, dans lequel le fluorophore est lié par covalence à la biomolécule.

3. Procédé selon la revendication 1 ou 2, dans lequel la biomolécule est un peptide.

4. Procédé selon la revendication 3, dans lequel le peptide est composé d'acides aminés protéinogènes.

5. Procédé selon l'une quelconque des revendications précédentes, dans lequel la biomolécule comprend un groupe thiol, notamment dans lequel la biomolécule comprend un résidu de cystéine, et le fluorophore est lié par covalence à la cystéine.

6. Procédé selon l'une quelconque des revendications précédentes, dans lequel le fluorophore est **caractérisé par** un clignotement à l'échelle de temps de la milliseconde.

7. Procédé selon l'une quelconque des revendications précédentes, dans lequel le fluorophore est une rhodamine de silicium, notamment une rhodamine de silicium hydroxyméthylique.

8. Procédé selon l'une quelconque des revendications précédentes, dans lequel la biomolécule est un peptide, et lesdits variants diffèrent du peptide en ce que concerne leur(s) :
a. séquence d'acides aminés,
b. modification post-traductionnelle de leur séquence d'acides aminés, notamment en ce qui concerne une modification post-traductionnelle sélectionnée parmi le groupe constitué d'une épimérisation, déamidation, citrullination, phosphorylation, ribosylation, glycosylation, acétylation, méthylation, hydroxylation ;
notamment dans lequel la modification post-traductionnelle est sélectionnée parmi le groupe constitué d'une épimérisation, déamidation et citrullination ;
c. changements conformationnels de la biomolécule en raison d'interactions avec d'autres biomolécules ou son environnement.

9. Procédé selon l'une quelconque des revendications précédentes, dans lequel la biomolécule est ancrée sur une surface, notamment une surface solide, une surface cellulaire ou une surface vésiculaire.
